# EUROPÄISCHE PATENTANMELDUNG

(11) **EP 0 888 787 A2**
(43) Veröffentlichungstag der Anmeldung: **07.01.1999**
(21) Anmeldenummer: 98108515.2
(22) Anmeldetag: 11.05.1998
(51) Int. Cl.: A61M 1/00, A61M 25/00, A61L 29/00

(54) **Medizintechnische Vorrichtung zur Verbesserung der Hautfixierung von Dauerkathetern und anderen transcutanen Implantaten bei reduzierter Infektionsgefahr**

(30) Priorität: 03.07.1997 DE 19728489
(71) Anmelder: HÜLS AKTIENGESELLSCHAFT, 45764 Marl (DE)
(72) Erfinder: Hämmerle, Hugo, Dr., 72072 Tübingen (DE); Schindler, Fritz, Dr., 45879 Gelsenkirchen (DE)

(57) **Zusammenfassung**

Die Erfindung betrifft transcutane, medizintechnische Vorrichtungen, wobei sich auf ihrer Oberfläche ein faserhaltiges Material mit freien, in natürlicher Struktur vorliegenden Kollagenfasern befindet.

Weiterhin betrifft die Erfindung Verfahren zur Aufbringung des faserhaltigen Materials auf die Oberfläche einer transcutanen, medizintechnischen Vorrichtung bzw. die Erzeugung des faserhaltigen Materials auf der Oberfläche der transcutanen, medizintechnischen Vorrichtung.

## Beschreibung

Die vorliegende Erfindung betrifft transcutane, medizintechnische Vorrichtungen sowie ein Verfahren zu ihrer Herstellung.

Transcutane medizintechnische Vorrichtungen sind über längere Zeit im Körper verbleibende hautdurchtretende Implantate wie beispielsweise Dauerkatheter.

Beispiele für transcutane Implantate sind Katheter für die Peritonealdialyse und Katheter für Langzeit-Perfüsionstheraphien. Bei der Langzeitanwendung dieser und anderer Implantate besteht die Gefahr der Infektion des Körperinneren mit Bakterien oder anderen Mikroorganismen: durch die unterschiedlichen Körperbewegungen werden temporär Zug- und Druckkräfte auf die Hautdurchgänge der Implantate ausgeübt, wodurch sich periodisch Spalten an der Grenzfläche Hautdurchgang/Hautgewebe bilden, durch die Mikroorganismen das Körperinnere infizieren können.

Aus der Literatur sind mehrere Vorschläge zur Fixierung von transcutanen Implantaten und Verhinderung von Infektionen bei transcutanen Vorrichtungen, wie z. B. Kathetern für die Peritonealdialyse unter Verwendung von Manschetten (englisch: cuff) bekannt.

Manschetten sind wenige mm bis wenige cm lange Hohlzylinder um die Katheter. Sie werden durch Überstreifen bzw. durch Aufkleben eines entsprechenden Bandes einzeln oder in Mehrzahl auf den Katheter gebracht. Aufgabe der Manschette ist es, mit ihrer äußeren Oberfläche einen engen Kontakt mit den, Körpergewebe einzugehen und so den Katheter zu fixieren und ein Einwandern von Mikroorganismen an der Grenzfläche Katheter/Körpergewebe zu verhindern. Zur Erreichung dieses Ziels besteht die Manschette an ihrer Außenseite gemäß US-PS 5 057 075 aus Dacron, einem als körperfreundlich angesehenen Material oder einem porösen Material (US-PS 5 308 338, US-PS 5 141 499), in das Körperzellen einwachsen können.

Als zusätzliche Maßnahme zur Verhinderung von mikrobiellen Infektionen des Körperinneren über die Hautdurchtrittsstelle werden Manschetten gelegentlich in Kombination mit antiseptischen Substanzen eingesetzt. US-PS 5 308 338 offenbart eine im Katheter verlaufende Röhre, durch die antiseptische Flüssigkeiten an das Manschettenmaterial abgegeben werden kann.

US-PS 5 049 140 beschreibt den Einsatz von antimikrobiellen Substanzen im Manschettenmaterial.

Weitere Patentschriften, die eine Fixierung und/oder Infektionsverhinderung im Zusammenhang mit der Anwendung transcutaner Katheter offenbaren, sind beispielsweise: US-PS 5 098 413, US-PS 5 057 075, US-PS 4 772 269, US-PS 4 687 471 und US-PS 4 623 329. Es werden vordringlich bestimmte geometrische Ausführungsformen von Kathetern für einzelne Anwendungsformen beschrieben.

Obwohl der Einsatz von Manschetten gemäß dem Stand der Technik die Zeitspanne, nach der Katheter aufgrund von Infektionserscheinungen gewechselt werden müssen verlängern kann, sind die Probleme der Fixierung und Infektionsvermeidung beim Einsatz transcutaner Vorrichtungen für den Langzeiteinsatz noch nicht zufriedenstellend gelöst. Insbesondere führt das Einwachsen von Körpergewebe in poröse Materialien nicht zu einer dauerhaften, das Vordringen von Infektionskeimen zuverlässig verhindernden Verbindung. Der Einsatz von antimikrobiellen und/oder antiseptischen Substanzen ist als störanfällige, schwer handhabbare Behelfsmaßnahme zu werten.

Aus einem anderen technischen Gebiet sind kollagenhaltige Verbundmaterialien als gut mit dem menschlichen Körper verwachsende Materialien bekannt (DE-PS 36 327 316. DE-AN 195 29 036.4).

Der vorliegenden Erfindung liegt die Aufgabe zugrunde, eine Fixierung von medizintechnischen Vorrichtungen im oder am Körper zu ermöglichen und dadurch eine Infektion des Körperinneren über die Körperdurchtrittsstelle zu verhindern.

Es wurde überraschenderweise gefunden, daß transcutane medizintechnische Vorrichtungen, die auf ihrer Oberfläche ein faserhaltiges Material mit freien in natürlicher Struktur vorliegenden Kollagenfasern aufweisen, eine mikrobielle Infektion des Körperinneren über die Hautdurchtrittsstelle der transcutanen medizintechnischen Vorrichtung in hohem Maße verhindern.

Das Körpergewebe verwächst infektions- und abstoßungsfrei mit den Kollagenfasern der transcutanen, medizintechnischen Vorrichtung und bildet so eine dauerhafte Einheit mit der transcutanen medizintechnischen Vorrichtung. Dadurch ist Infektionskeimen der Weg ins Körperinnere versperrt.

Gegenstand der vorliegenden Erfindung ist daher eine transcutane, medizintechnische Vorrichtung, dadurch gekennzeichnet, daß sich auf ihrer Oberfläche ein faserhaltiges Material mit freien, in natürlicher Struktur vorliegenden Kollagenfasern befindet.

Weiterhin ist Gegenstand der vorliegenden Erfindung ein Verfahren zur Herstellung einer transcutanen medizintechnischen Vorrichtung, dadurch gekennzeichnet, daß auf ihre Oberfläche ein faserhaltiges Material mit freien, in natürlicher Struktur vorliegenden Kollagenfasern aufgebracht wird.

Eine bevorzugte Ausführungsform der vorliegenden Erfindung ist eine transcutane medizintechnische Vorrichtung, erhältlich durch Erzeugung eines faserhaltigen Materials unter
- Herstellung eines Kollagenfilzes aus in natürlicher Struktur vorliegenden Kollagenfasern,
- Infiltration dieses Filzes mit einer polymerisierbaren Monomermischung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel
und anschließendes Aufbringen des so erzeugten faserhaltigen Materials auf die Oberfläche der transcutanen, medizintechnischen Vorrichtung.

Weiterhin beschreibt die vorliegende Erfindung ein Verfahren zur Herstellung einer transcutanen, medizintechnischen Vorrichtungen, dadurch gekennzeichnet, daß das faserhaltige Material durch
- Herstellung eines Kollagenfilzes aus in natürlicher Struktur vorliegenden Kollagenfasern,
- Infiltration dieses Filzes mit einer polymerisierbaren Monomermischung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel
erzeugt und anschließend auf die Oberfläche der transcutanen, medizintechnischen Vorrichtung aufgebracht wird.

Das faserhaltige Material ist ein kollagenhaltiger Verbundwerkstoff, dessen Oberfläche ein Filz aus freien, in natürlicher Struktur vorliegenden Kollagenfasern bildet. Zur Herstellung dieses Kollagen/Polymer-Verbundmaterials wird zunächst gemäß der Deutschen Patentanmeldung "Biokompatibles Verbundmaterial und Verfahren zu seiner Herstellung" (DE-AN 195 29.036.4) ein Filz aus freien, in natürlicher Struktur vorliegenden Kollagenfasern gewonnen und anschließend mit einer polymerisierbaren Monomerpräparation infiltriert. Enthält die Monomerenpräparation keine Polymerisationsinitiatoren, wird die Polymerisation z. B. strahleninduziert gestartet. In allen Fällen muß die Polymerisation oberflächengequencht durchgeführt werden. Man erhält auf diese Weise einen kollagenhaltigen Verbundwerkstoff mit einer nicht auspolymerisierten Schicht an der Oberfläche. Diese Schicht wird zur Exponierung der Kollagenfasern mit einem geeigneten Lösemittel abgelöst. Über die Wahl der Quenchparameter läßt sich die Dicke und Beschaffenheit der während der Polymerisation nicht aushärtenden Oberflächenschicht beeinflussen.

Eine weitere Ausführungsform der vorliegenden Erfindung ist eine transcutane, medizintechnische Vorrichtung, erhältlich durch Aufbringen eines faserhaltigen Materials unter
- Herstellung eines Kollagenfilzes aus in natürlicher Struktur vorliegenden Kollagenfasern,
- Infiltration dieses Filzes mit einer polymerisierbaren Monomermischung,
- Aufbringen des so infiltrierten Filzes auf die Oberfläche der transcutanen, medizintechnischen Vorrichtung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltene Polymerisats zur Exponierung der Kollagenfasern durch Einsatz geeigneter Lösemittel
auf die Oberfläche der transcutanen, medizintechnischen Vorrichtung.

Neben der Aufbringung des faserhaltigen Materials auf die transcutane medizintechnische Vorrichtung kann dieses auch direkt auf der Oberfläche der transcutanen, medizintechnischen Vorrichtung erzeugt werden.

Ein solches Verfahren wird durchgeführt, in dem das faserhaltige Material durch
- Herstellung eines Kollagenfilzes aus in natürlicher Struktur vorliegenden Kollagenfasern,
- Infiltration dieses Filzes mit einer polymerisierbaren Monomermischung,
- Aufbringen des so infiltrierten Filzes auf die Oberfläche der transcutanen medizintechnischen Vorrichtung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel
auf die Oberfläche der transcutanen, medizintechnischen Vorrichtung aufgebracht wird.

### Detaillierte Beschreibung der Erfindung

Kern der vorliegenden Erfindung ist der Einsatz spezieller Materialien bei der Herstellung von transcutanen medizintechnischen Vorrichtungen. Diese Kollagen/Polymer-Verbundmaterialien haben an ihrer Oberfläche freie, in natürlicher Struktur vorliegende Kollagenfasern, die vermutlich für das überraschend schnelle und vollständige Einheilen bzw. Verwachsen dieser Materialien mit Körpergewebe - wie zum Beispiel Hautbindegewebe - verantwortlich sind. Transcutane medizintechnische Vorrichtungen, die an speziellen Stellen diese Kollagen/Polymer-Verbundmaterialien, z. B. in Form von Manschetten, enthalten, verwachsen über diese Materialien mit dem Körpergewebe, was zu einer Fixierung der transcutanen Vorrichtung am bzw. im Körper führt und - wenn sich diese Kollagen/Polymer-Verbundmaterialien am Körperdurchtritt der transcutanen Vorrichtung befinden - mikrobielle Infektionskeime am Einwandern in das Körperinnere hindert (siehe Abb. 1).

### Einsatz eines feinfaserigen Kollagens

Die Eigenschaft des erfindungsgemäßen Implantatmaterials, mit der Haut zu verwachsen, resultiert aus den feinen, im Implantatmaterial verankerten Kollagenfasern, die aus der Implantatoberfläche herausragen. Diese Fasern haben einen Durchmesser bis zu 500, vorzugsweise von ca. 200 nm und entsprechen in ihrer Struktur dem natürlichen Kollagen (siehe Abb. 2). Die von menschlichen Zellen gebildeten Kollagenfasern können sich daher gut mit den Kollagenfasern des Verbundmaterials verbinden und sorgen für eine günstige Einheilung der transcutanen medizintechnischen Vorrichtungen mit dem Körpergewebe.

Die üblichen, kommerziell erhältlichen Kollagenpräparate sind mehr oder weniger stark unter Aufgabe ihrer nativen Struktur denaturiert.

Zur Herstellung von feinfaserigem Kollagen mit natürlicher Struktur läßt sich folgendes Verfahren anwenden: Kollagen, z. B. aus Rinder- oder Rattenschwanzsehnen, wird in verdünnter Essigsäure gelöst und anschließend durch Dialyse und Zentrifugation gereinigt. Der die Kollagenmoleküle enthaltende Zentrifugenüberstand wird danach abgenommen und in sterile Gefäße überführt. Durch Änderung des pH-Werts und der Salzkonzentration organisieren sich die Kollagenmoleküle zu filzartigen Matten aus feinfaserigem Kollagen.

### Herstellung der Kollagen/Polymer-Verbundmaterialien

Ausschlaggebend für den Applikationserfolg eines kollagenhaltigen Verbundwerkstoffes ist die Exposition der nativen Kollagenfasern an der Oberfläche bei der Herstellung.

Dies wird bei dem hier beschriebenen Werkstoff erreicht durch Tränken der Kollagenmatten mit aushärtbaren Monomeren, z. B. Methylmethacrylat (MMA), darauffolgende radikalische Polymerisation (Aushärtung) in Anwesenheit von der Oberfläche der getränkten Kollagenmatten her wirkender Polymerisationsinhibitoren und anschließende Entfernung der obersten Schicht des Kollagen/Polymer-Verbundmaterials zur partiellen Freilegung der Kollagenfasern (exponierter Filz).

Vorzugsweise wird ein Verfahren verwendet, das auf dem Prinzip der Inhibierung der Polymerisationsreaktion an der Oberfläche durch Sauerstoff beruht. Die mit einer Monomerenmischung getränkten Kollagenmatten werden bei diesem Verfahren nicht in abgeschlossenen Formen, sondern unter Hinzutritt von Sauerstoff bzw. eines Sauerstoff-haltigen Gasgemisches auspolymerisiert. Da Polymerisationsreaktionen durch Sauerstoff inhibiert werden können, verbleibt an der Oberfläche der Proben eine nicht ausgehärtete äußere Schicht, die anschließend durch Behandlung mit einem geeigneten Lösungsmittel, z. B. Aceton, entfernt wird. Auf diese Weise lassen sich an der Oberfläche die Kollagenfasern freilegen. Die Dicke der Schicht kann durch Wahl der entsprechenden Parameter (Sauerstoffkonzentration, Dauer des Aushärtungsvorganges, Lichtintensität, Temperatur, Lösungsmittel) gesteuert werden.

Als aushärtbare Monomere können einzeln (Homopolymerisate) oder in Kombination mit anderen Monomeren (Copolymerisate) prinzipiell alle Substanzen eingesetzt werden, die über eine Radikalreaktion polymerisieren, wie beispielsweise Styrol, Vinylverbindungen, Maleinsäureanhydrid oder Acrylsäure- und Methacrylsäurealkylester, wobei die Alkylgruppe 1 bis 12 C-Atome enthalten kann. Die Struktur kann linear, verzweigt, cycloaliphatisch, aromatisch oder substituiert aromatisch sein. Weiterhin einsetzbar sind heterozyklische Monomere, die entweder Stickstoff, Schwefel oder Sauerstoff in der Seitenkette tragen. Die Monomere können als Einzelkomponenten oder in Form von Monomermischungen bzw. Monomer/Polymermischungen mit oder ohne Füllstoffe eingesetzt werden.

Die polymerisierbare Monomermischung kann radikalisch polymerisierbare Monomere, bevorzugt Acrylate, besonders bevorzugt Methacrylate enthalten.

Die polymerisierbare Monomerenmischung kann weiterhin eine oder mehrere Verbindungen aus der folgenden Gruppe enthalten: Methylmethacrylat, Ethylmethacrylat, n-Butylmethacrylat, Isobutylmethacrylat, 2-Ethylhexylmethacrylat, Cyclohexylmethacrylat, Isobornylmethacrylat, Tetrahydrofurfurylmethacrylat, Benzylmethacrylat, Morpholinoethylmethacrylat, Diethylenglycol-dimethacrylat, Triethylenglycol-dimethacrylat, Diurethandimethacrylat (Umsetzungsprodukt aus Trimethylhexamethylendiisocyanat mit zwei Mol 2-Hydroxyethylmethacrylat) Isopropyliden-bis(2(3)-hydroxy-3(2)-(4-phenoxy)-propylmethacrylat) und/oder Methacrylsäure.

Außerdem kann die polymerisierbare Monomerenmischung ein oder mehrere Verbindungen aus der folgenden Gruppe enthalten: Styrol, α-Methylstyrol, Styrolsulfonsäure, Vinylverbindungen und/oder Maleinsäureanhydrid.

Vinylverbindungen können Ethylen, Propylen oder Butylene, aber auch Vinylchlorid oder Butadien sein. Weitere Bestandteile der Monomerenmischung können Lösungsmittel und/oder Füllstoffe sowie Polymerisationsinitiatoren sein.

Als Polymerisationsinitiatoren lassen sich u. a. Azonitrile, Alkylperoxide, Acylperoxide, Hydroperoxide, Peroxoketone, Perester und Peroxocarbonate, Peroxodisulfat, Persulfat sowie alle üblichen Photoinitiatoren verwenden.

Die Polymerisationsinitiierung kann ebenfalls thermisch oder durch elektromagnetische Strahlung wie z. B. UV-Licht oder γ-Strahlung erfolgen.

Zur Erzeugung einer nicht polymerisierten Schicht auf der Oberfläche des Kollagen-Verbundwerkstoffs wird die Polymerisation in Anwesenheit von Sauerstoff oder eines Sauerstoff-haltigen Gasgemisches durchgeführt. Die so erzeugte, nicht durchpolymerisierte Schicht wird mit einem geeigneten Lösemittel, wie z. B. Aceton Methylethylketon, Acetonitril oder THF, abgelöst.

Es gibt mehrere Möglichkeiten, auf diese Weise erhaltene Materialien mit freien Kollagenfasern an der Oberfläche von transcutanen medizintechnischen Vorrichtungen erfindungsgemäß anzubringen:
- Aufbringung des noch plastischen Materials, d. h. einer Mischung von Monomeren und Kollagen auf eine medizintechnische Vorrichtung mit anschließender oberflächengequenchter Aushärtung und Ablösung der nicht durchpolymerisierten Oberfläche,
- Herstellung von Hohlzylindern, beispielsweise durch Infiltration des Kollagenfilzes im Wandraum einer Form. Das hochviskose Monomer/Kollagen-Material wird dann aus der Form genommen, auf einen Dom gespießt und oberflächengequencht in Sauerstoff bzw. einem Sauerstoff-haltigen Gasgemisch polymerisiert. Man erhält nach Ablösung der nicht durchpolymerisierten Außenschicht mit einem Lösungsmittel auf diesem Weg Hohlzylinder aus dem Polymer/Kollagen-Verbundmaterial mit einer glatten Innenseite und einer Außenseite mit freien Kollagenfasern. Durch Wahl unterschiedlicher Formen lassen sich Hohlzylinder mit unterschiedlichen Abmessungen herstellen. Vorzugsweise werden Formen verwendet mit:
- einer Länge von 0.2 bis 7 cm
- einem Außendurchmesser von 0.5 bis 2 cm
- einem Innendurchmesser von 0.2 bis 1.5 cm.

Auf diesem oder einem anderen Weg hergestellte Hohlzylinder mit freien Kollagenfasern aufder Außenseite lassen sich in unterschiedlichen Ausführungsformen zur Fixierung von transcutanen Implantaten einsetzen, beispielsweise als:
- Manschette, die über den Katheter gezogen und an gewünschter Stelle durch Ringspannungskräfte, Einsatz eines biokompatiblen Klebers wie z. B. Histoacrylkleber oder anderweitig fixiert wird,
- als Hülse, die über Steckanschlüsse in den Katheter gefügt wird,
- als Hülse, die vertikal in der Haut fixiert wird und einige Wochen Zeit bekommt, in das Hautgewebe einzuwachsen. Danach wird ein Katheter durch die Hülse geschoben und der Ringspalt zwischen Katheter und der Innenwand der Hülse mit Silikonöl oder einem anderen geeigneten Material abgedichtet.

Zur Unterstützung des Einwachsvorgangs bei der erfindungsgemäßen Anwendung der Kollagen/Polymer-Verbundmaterialien bei transcutanen medizintechnischen Vorrichtungen kann die Oberfläche der transcutanen Vorrichtung kurz vor ihrer Anwendung mit einem Gel, das Antibiotika, Gewebewachstumfördernde Stoffe und/oder andere Wirkstoffe enthalten kann, bestrichen werden. Zur Bereitung dieses Gels wird eine wässrige Lösung oder Suspension der Wirkstoffe bereitet, die dann durch Zusatz gelbildender Substanzen verfestigt wird.

### Inhaltsstoffe des Gels

### Antibiotika

Antibiotika werden bei der Gelbildung in einer Menge eingesetzt, die unter Berücksichtigung der Diffusionsverluste eine Sepsis über mehrere Tage verhindert. Besonders geeignete Antibiotika sind Penicillin, Streptomycin sowie weitere, vornehmlich lipophile Antibiotika.

### Wachstumsfaktoren

Es werden ausreichende Mengen eines handelsüblichen epidermalen Wachstumsfaktors oder andere Faktoren, wie z. B. der Fibroblast Growth Factor oder der Platelet Derived Growth Factor zugesetzt. Es kann vorteilhaft sein, zusammen mit den Wachstumsfaktoren Dextran oder Calciumphosphat zuzusetzen, um eine Retardwirkung zu erzielen (EP-PS 530 458, JP-PS 6 310 5765).

### Ausbildung des Gels

Die die Wirkstoffe enthaltende wässrige Lösung oder Suspension kann auf mehreren Wegen in ein Gels überführt werden:
- Zusatz von Fibrinogen, Thrombin und Aprotinin
- Zusatz von Kollagen nach Parson-Wingerter und Saltzman (Biotechnol. Prog. 9, S. 600 - 607/1993).
- Zusatz einer Mischung aus Kollagen und Calciumphosphat
- Zusatz von Natriumalginat und anschließende Auslösung der Gelbildung durch Zugabe von CaCl₂.

Das folgende Beispiel soll die Erfindung näher erläutern:

18 zylinderförmige Probekörper (Durchmesser: 4 mm, Länge: 15 mm) wurden 3 jungen männlichen Ratten dorsal in zwei Reihen subcutan von cranial nach kaudal implantiert. Diese 18 Probekörper bestanden aus:
- 3 x Polymethylmethacrylat (PMMA)
- 6 x PMMA/Kollagen-Verbundmaterial, Kollagenfasern nicht quervernetzt
- 6 x PMMA/Kollagen-Verbundmaterial, Kollagenfasern mit Glutardialdehyd vernetzt
- 3 x Polytetrafluorethylen

Die Kollagen-haltigen Probekörper wurden wie folgt hergestellt: in natürlicher Struktur vorliegendes Kollagen wurde mit einer Methylmethacrylat-Monomermischung (siehe DE-AN 195 29.036.4) infiltriert und anschließend in einer wannenförmigen Aushärtform unter einer Halogenlampe mit einem Strahlungsmaximum bei 340 nm für 10 min ausgehärtet. Diese Aushärtung wurde in einem Exsikkator durchgeführt, der zuvor mit Sauerstoff geflutet worden war Danach wurden die Proben 10 min unter Bewegung in Aceton zur Ablösung der nicht ausgehärteten Oberflächenschicht getaucht. Zur Herstellung der Probekörper mit quervernetztem Kollagen wurde das in natürlicher Struktur vorliegende Kollagen vor Infiltration mit der Monomermischung mit Glutardialdehyd fixiert.

Da während der Aushärtung der Sauerstoff lediglich zu dem Teil der Probenoberfläche Zutritt hatte, der in der wannenförmigen Aushärtform oben lag, wurden bei der anschließenden Acetonbehandlung freie Kollagenfasern lediglich an dieser Teiloberfläche (knapp 50 % der Gesamtoberfläche) exponiert.

Alle Probekörper heilten abstoßungs- und endzündungsfrei in das Hautgewebe ein. Jedoch zeigten sich bei der nach 28 Tagen vorgenommenen Bestimmung der zur Extraktion der Implantate aus dem Hautgewebe der abgetöteten Ratten erforderlichen Zugkräfte deutliche Unterschiede:

| Material | Durchschnitt der zur Extraktion der Probekörper aufgewendeten Zugkräfte [N] |
|---|---|
| Polytetrafluorethylen | < 1,0 |
| PMMA | 3,0 |
| PMMA/Kollalgen-Verbundwerkstoff, nicht quervernetzt | 6,5 |
| PMMA/Kollagen-Verbundwerkstoff, quervernetzt | 5,7 |

Die bei den kollagenhaltigen Verbundmaterialien zur Extraktion der Probekörper aufzuwendenen Kräfte zeigen die gute Verbindung der Probenkörper mit dem Körpergewebe auf zumal die freien Kollagenfasern lediglich auf knapp 50 % der Gesamtoberfläche der Probenkörper exponiert werden.

## Patentansprüche

1. Transcutane medizintechnische Vorrichtung,
dadurch gekennzeichnet,
daß sich auf ihrer Oberfläche ein faserhaltiges Material mit freien, in natürlicher Struktur vorliegenden Kollagenfasern befindet.

2. Transcutane medizintechnische Vorrichtung nach Anspruch 1,
erhältlich durch Erzeugung eines faserhaltigen Materials unter
- Herstellung eines Kollagenfilzes aus in natürlicher Struktur vorliegenden Kollagenfasern,
- Infiltration dieses Filzes mit einer polymerisierbaren Monomermischung,
- Durchführung einer radikalischen Polymerisation in Anwesenheit von der Oberfläche des infiltrierten Filzes her wirkenden Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel und
anschließendem Aufbringen des so erzeugten faserhaltigen Materials auf die Oberfläche der transcutanen, medizinischen Vorrichtung.

3. Transcutane medizintechnische Vorrichtung nach Anspruch 1,
erhältlich durch Aufbringen eines faserhaltigen Materials unter
- Herstellung eines Kollagenfilzes aus in natürlicher Struktur vorliegenden Kollagenfasern,
- Infiltration dieses Filzes mit einer polymerisierbaren Monomermischung,
- Aufbringen des so infiltrierten Filzes auf die Oberfläche der transcutanen, medizintechnischen Vorrichtung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel
auf die Oberfläche der transcutanen, medizintechnischen Vorrichtung.

4. Transcutane medizintechnische Vorrichtungen nach den Ansprüchen 1 bis 3,
dadurch gekennzeichnet,
daß die polymerisierbare Monomerenmischung radikalisch polymerisierbare Monomere enthält.

5. Transcutane medizintechnische Vorrichtungen nach den Ansprüchen 1 bis 4,
dadurch gekennzeichnet,
daß die polymerisierbare Monomermischung Acrylate enthält.

6. Transcutane medizintechnische Vorrichtungen nach den Ansprüchen 1 bis 4,
dadurch gekennzeichnet,
daß die polymerisierbare Monomermischung Methacrylate enthält.

7. Transcutane medizintechnische Vorrichtung nach den Ansprüchen 1 bis 4,
dadurch gekennzeichnet,
daß die polymerisierbare Monomermischung eine oder mehrere Verbindungen aus der folgenden Gruppe enthält:
Methylmethacrylat, Ethylmethacrylat, n-Butylmethacrylat, Isobutylmethacrylat, 2-Ethylhexylmethacrylat, Cyclohexylmethacrylat, Isobornylmethacrylat, Tetrahydrofurfurylmethacrylat, Benzylmethacrylat, Morpholinoethylmethacrylat, Diethylenglycol-dimethacrylat, Triethylenglycol-dimethacrylat, Diurethandimethacrylat (Umsetzungsprodukt aus Trimethylhexamethylendiisocyanat mit zwei Mol 2-Hydroxyethylmethacrylat), Isopropyliden-bis(2(3)-hydroxy-3(2)-(4-phenoxy)-propylmethacrylat) und/oder Methacrylsäure.

8. Transcutane medizintechnische Vorrichtung nach den Ansprüchen 1 bis 4,
dadurch gekennzeichnet,
daß die polymerisierbare Monomermischung eine oder mehrere Verbindungen aus der folgenden Gruppe enthält:
Styrol, α-Methylstyrol, Styrolsulfonsäure, Vinylverbindungen und/oder Maleinsäureanhydrid.

9. Verfahren zur Herstellung einer transcutanen, medizintechnischen Vorrichtung,
dadurch gekennzeichnet,
daß auf ihre Oberfläche ein faserhaltiges Material mit freien, in natürlicher Struktur vorliegenden Kollagenfasern aufgebracht wird.

10. Verfahren nach Anspruch 9,
dadurch gekennzeichnet,
daß das faserhaltige Material durch
- Herstellung eines Kollagenfilzes aus in natürlicher Struktur vorliegenden Kollagenfasern,
- Infiltration dieses Filzes mit einer polymerisierbaren Monomermischung,
- Durchführung einer radikalischen Polymerisation in Anwesenheit von der Oberfläche des infiltrierten Filzes her wirkender Polymerisationsinhibitoren
- Ablösen der nicht durchpolymerisierten Oberflächenschicht des so erhaltenen Polymerisats zur Exponierung der Kollagenfasern durch Einsatz geeigneter Lösemittel
erzeugt und anschließend auf die Oberfläche der transcutanen, medizinischen Vorrichtung aufgebracht wird.

11. Verfahren nach Anspruch 9,
dadurch gekennzeichnet,
daß das faserhaltige Material durch
- Herstellung eines Kollagenfilzes aus in natürlicher Struktur vorliegenden Kollagenfasern,
- Infiltration dieses Filzes mit einer polymerisierbaren Monomermischung,
- Aufbringen des so infiltrierten Filzes auf die Oberfläche der transcutanen, medizintechnischen Vorrichtung,
- Durchführung einer radikalischen Polymerisation in Anwesenheit von der Oberfläche des infiltrierten Filzes her wirkender Polymerisationsinhibitoren
- Ablösung der nicht durchpolymerisierten Oberflächenschicht des so erhaltenen Polymerisats zur Exponierung der Kollagenfasern durch Einsatz geeigneter Lösemittel
auf die Oberfläche der transcutanen, medizintechnischen Vorrichtung aufgebracht wird.

12. Verfahren nach den Ansprüchen 9 bis 11,
dadurch gekennzeichnet,
daß die polymerisierbare Monomerenmischung radikalisch polymerisierbare Monomere enthält.

13. Verfahren nach den Ansprüchen 9 bis 11,
dadurch gekennzeichnet,
daß die polymerisierbare Monomermischung Acrylate enthält.

14. Verfahren nach den Ansprüchen 9 bis 11,
dadurch gekennzeichnet,
daß die polymerisierbare Monomermischung Methacrylate enthält.

15. Verfahren nach den Ansprüchen 9 bis 11,
dadurch gekennzeichnet,
daß die polymerisierbare Monomermischung eine oder mehrere Verbindungen aus der folgenden Gruppe enthält:
Methylmethacrylat, Ethylmethacrylat, n-Butylmethacrylat, Isobutylmethacrylat, 2-Ethylhexylmethacrylat, Cyclohexylmethacrylat, Isobornylmethacrylat, Tetrahydrofurfurylmethacrylat, Benzylmethacrylat, Morpholinoethylmethacrylat, Diethylenglycol-dimethacrylat, Triethylenglycol-dimethacrylat, Diurethandimethacrylat (Umsetzungsprodukt aus Trimethylhexarnethylendiisocyanat mit zwei Mol 2-Hydroxyethylmethacrylat), Isopropyliden-bis(2(3)-hydroxy-3(2)-(4-phenoxy)-propylmethacrylat) und/oder Methacrylsäure.

16. Verfahren nach den Ansprüchen 9 bis 11,
dadurch gekennzeichnet,
daß die polymerisierbare Monomermischung eine oder mehrere Verbindungen aus der folgenden Gruppe enthält:
Styrol, α-Methylstyrol, Styrolsulfonsäure, Vinylverbindungen und/oder Maleinsäureanhydrid.
